(19) Europäisches Patentamt
European Patent Office
Office européen des brevets

(11) Veröffentlichungsnummer: **0 049 797**
**A2**

(12) **EUROPÄISCHE PATENTANMELDUNG**

(21) Anmeldenummer: **81107681.9**

(22) Anmeldetag: **28.09.81**

(51) Int. Cl.³: **A 61 K 31/415**
//C07D233/22

(30) Priorität: **10.10.80 DE 3038395**

(43) Veröffentlichungstag der Anmeldung:
**21.04.82 Patentblatt 82/16**

(84) Benannte Vertragsstaaten:
**CH DE LI**

(71) Anmelder: **BAYER AG**
**Zentralbereich Patente, Marken und Lizenzen**
**D-5090 Leverkusen 1, Bayerwerk(DE)**

(72) Erfinder: **Enders, Edgar, Dr.**
**René-Bohn-Strasse 16**
**D-5000 Koeln 80(DE)**

(72) Erfinder: **Andrews, Peter, Dr.**
**Gellertweg 2**
**D-5600 Wuppertal 1(DE)**

(54) **2-(2,3-Dimethyl-phenoxymethyl)-2-imidazolin enthaltendes Anthelmintikum.**

(57) Die Erfindung betrifft 2-(2,3-Trimethyl-phenoxymethyl)-2-imidazolin der allgemeinen Formel I

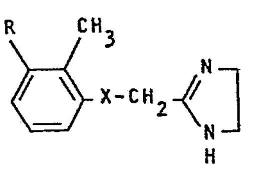

1

in der R und X die in der Beschreibung angegebene Bedeutung haben, zur Verwendung von bei der Bekämpfung von parasitären Helminthen bei Mensch und Tier, 2-(2,3-Dimethylphenoxy)-2-imidazolin enthaltende Arzneimittel und deren Herstellung.

EP 0 049 797 A2

0049797

BAYER AKTIENGESELLSCHAFT 5090 Leverkusen, Bayerwerk
Zentralbereich
Patente, Marken und Lizenzen Ad/kl-c

# 2-(2,3-Dimethyl-phenoxymethyl)-2-imidazolin enthaltendes Anthelmintikum

Die Erfindung betrifft 2-(2,3-Trimethyl-phenoxymethyl)-2-imidazolin zur Verwendung von bei der Bekämpfung von parasitären Helminthen bei Mensch und Tier, 2-(2,3-Dimethylphenoxy)-2-imidazolin enthaltende Arzneimittel und deren Herstellung.

Es ist bereits bekannt, daß Imidazolinderivate beispielsweise der allgemeinen Formel I

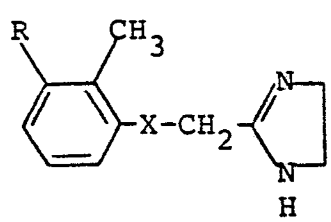

I

in der

R für Methyl oder Chlor und
X für 0 oder NH steht,

als Schädlingsbekämpfungsmittel gegen Ektoparasiten, insbesondere Arthropoden, z.B. Zecken, sowie Mikroorganismen wie phytopathogene Pilze verwendet werden können (s. z.B. die DE-OSen 2 750 902 und 2 756 638 sowie das belgische Patent 866 513).

Es wurde nun überraschend gefunden, daß die Verbindung 2-(2,3-Dimethyl-phenoxymethyl)-2-imidazolin mit der Formel

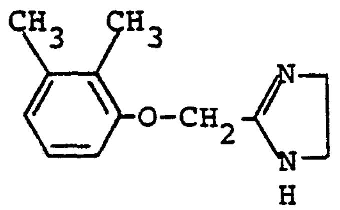

eine ausgezeichnete Wirkung gegen parasitäre Helminthen bei Mensch und Tier bei gleichzeitig geringer Toxizität besitzt.

Die Erfindung betrifft daher 2-(2,3-Dimethyl-phenoxymethyl)-2-imidazolin zur Verwendung bei der Bekämpfung von parasitären Helminthen bei Mensch und Tier.

Die genannte Verbindung, ihre Synthese sowie ihre Säureadditionssalze sind aus den oben angegebenen Patentschriften bekannt. Bevorzugte Synthesewege ergeben sich beispielsweise aus den nachfolgenden Formelgleichungen:

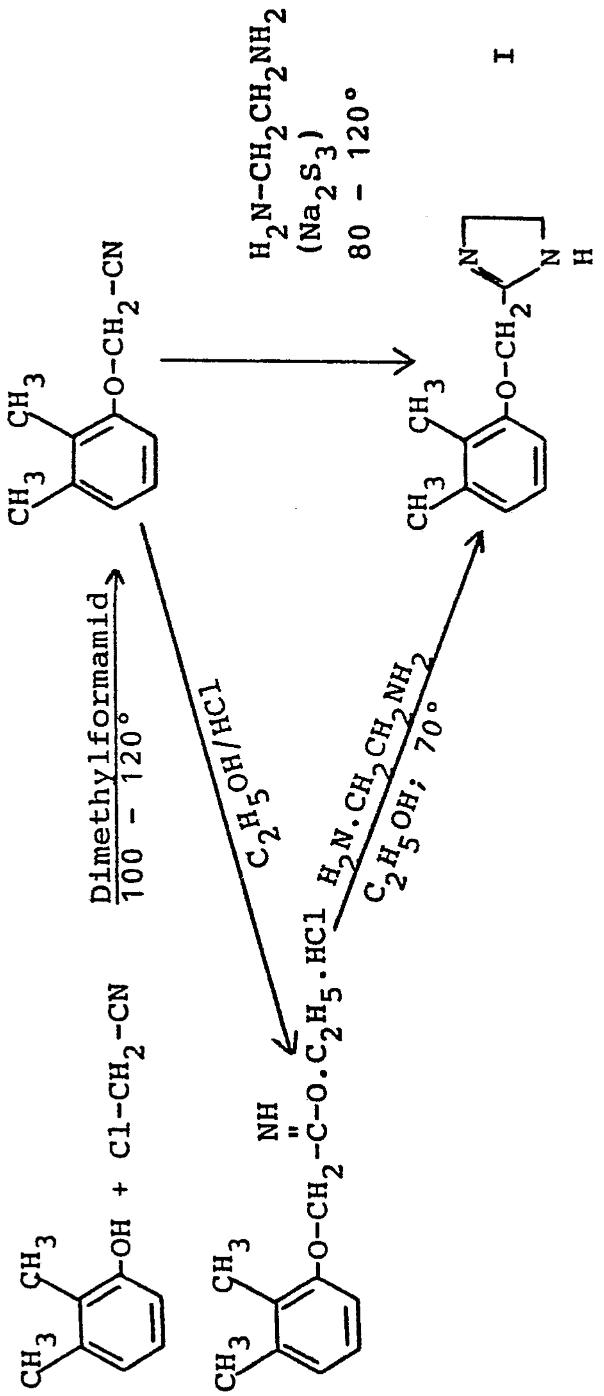
Dimethylformamid
100 - 120°
$C_2H_5OH/HCl$
$H_2N.CH_2CH_2NH_2$
$C_2H_5OH$; 70°
$H_2N-CH_2CH_2NH_2$
($Na_2S_3$)
80 - 120°

I

Als Säureadditionssalze des 2-(2,3-Dimethyl-phenoxymethyl)-2-imidazolins kommen vorzugsweise solche in Betracht, die toxikologisch annehmbar sind, beispielsweise Salze des Chlorwasserstoffs, der Schwefelsäure, Phosphorsäure, Salpetersäure, Essigsäure, Methansulfonsäure, p-Toluolsulfonsäure, Naphthalin-1,5-disulfonsäure, Bernsteinsäure, Maleinsäure, Fumarsäure, Citronensäure, Benzoesäure, des 1,1'-Methylen-bis-(2-hydroxy-3-carboxy)-naphthalins.

Die Wirkung wurde im Tierversuch nach oraler, subcutaner und dermaler Applikation bei stark mit Parasiten befallenen Versuchstieren geprüft. Die angewendeten Dosierungen wurden sehr gut von den Versuchstieren vertragen.

Die neuen Anthelmintika können als Anthelmintika sowohl in der Human- als auch in der Veterinärmedizin verwendet werden.

Die neuen Anthelmintika können in bekannter Weise in die üblichen Formulierungen übergeführt werden.

Beispiel

Magen- und Darmwurmtest/Schaf

Experimentell mit Haemonchus contortus infizierte Schafe wurden nach Ablauf der Präpatenzzeit der Parasiten behandelt. Die Wirkstoffmenge wurde als reiner Wirkstoff in Gelatinekapseln oral appliziert oder in einem geeigneten Lösungsmittel gelöst und subcutan oder dermal appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren können (Dosis effectiva). Geprüfte Wirkstoffe und wirksame Dosierungen (dosis effectiva minima) sind aus der nachfolgenden Tabelle ersichtlich.

Die Verbindung zeigt eine überraschend gute und breite Wirkung gegen folgende Helminthen:

Hakenwürmer (z.B. Uncinaria stenocephala, Ancylostoma caninum, Bunostomum trigonocephalum), Trichostrongyliden (z.B. Nippostrongylus muris, Haemonchus contortus, Trichostrongylus colubriformis, Ostertagia circumcincta), Strongyliden (z.B. Oesophagostomum columbianum), Rhabditiden (z.B. Strongyloides ratti), Spulwürmer (z.B. Ascaris suum, Toxocara canis, Toxascaris leonina), Madenwürmer (z.B. Aspiculuris tetraptera), Heterakiden (z.B. Heterakis spumosa), Peitschenwürmer (z.B. Trichuris muris), Filarien (z.B. Litomosoides carinii, Dipetalonema witei), Cestoden (z.B. Hymenolepis nana, Taenia pisiformis, Echinococcus multilocularis) und Trematoden (z.B. Schistosoma mansoni, Fasciola hepatica).

Die Verbindung ist vor allem wirksam gegen Magen- und Darm-Nematoden der Wiederkäuer und besonders auch solcher, die gegen die gebräuchlichen Benzimidazol-Anthelmintika resistent und damit nicht mehr ausreichend therapierbar sind.

Die Verbindung wirkt nicht nur nach oraler oder parenteraler Verabreichung, sondern auch gut nach dermaler Applikation (Pour-On, Spot-On). Damit werden Verabreichungsweisen möglich, die bei den im Handel befindlichen nur nach oraler Gabe wirkenden Benzimidazol-Anthelmintika nicht möglich sind, andererseits aber für den Anwender vorteilhaft sind wie z.B. die dermale oder subcutane Behandlung.

0049797

Tabelle

| Wirkstoff | Applikationsweise | Dosis effectiva minima (Red. 90 % in mg/kg) Haemonchus contortus normaler Stamm | gegen Benzimidazole resistenter Stamm |
|---|---|---|---|
| 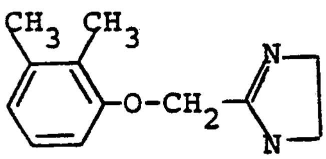 | per os | 0,5 | 0,5 |
| | subcutan | 0,5 | 0,5 |
| | dermal | 1 | 1 |
| Vergleichsverbindungen | | | |
| Thiabenzol | per os | 25 | 250 |
| Parbendazol | per os | 1 | 10 |
| Albendazol | per os | 0,5 | 5 |

Patentansprüche

1. 2-(2,3-Dimethyl-phenoxymethyl)-2-imidazolin zur Verwendung bei der Bekämpfung von parasitären Helminthen bei Mensch und Tier.

2. Arzneimittel enthaltend 2-(2,3-Dimethyl-phenoxymethyl)-2-imidazolin.

3. Anthelminthisches Arzneimittel enthaltend 2-(2,3-Dimethyl-phenoxymethyl)-2-imidazolin.

4. Verwendung von 2-(2,3-Dimethyl-phenoxymethyl)-2-imidazolin zur Herstellung von Arzneimitteln.

5. Verwendung von 2-(2,3-Dimethyl-phenoxymethyl)-2-imidazolin zur Bekämpfung von parasitären Helminthen bei Mensch und Tier.

6. Verfahren zur Behandlung von Erkrankungen, die durch parasitäre Helminthen hervorgerufen werden, dadurch gekennzeichnet, daß man 2-(2,3-Dimethyl-phenoxymethyl)-2-imidazolin Menschen oder Tieren im Bedarfsfall appliziert.